# EUROPEAN PATENT APPLICATION

(11) **EP 4 700 019 A1**
(43) Date of publication of application: **25.02.2026**
(21) Application number: 24792106.7
(22) Date of filing: 19.04.2024
(51) Int. Cl.: C07D 401/04, A61K 31/551, A61P 25/00, A61P 25/18, A61P 25/16, A61P 25/28, A61P 25/22, A61P 25/24

(54) **CRYSTAL FORM OF 1,5-DIHYDRO-2,4-BENZODIAZEPINE-3-ONE DERIVATIVE CITRATE, PREPARATION METHOD, AND USE THEREOF**

(30) Priority: 21.04.2023 CN 202310431178
(71) Applicant: Jiangsu NHWA Pharmaceutical Co., Ltd, Xuzhou, Jiangsu 221000 (CN)
(72) Inventor: DOU, Fei, Xuzhou, Jiangsu 221000 (CN); WANG, Shuo, Xuzhou, Jiangsu 221000 (CN); QIU, Yinli, Xuzhou, Jiangsu 221000 (CN); YANG, Qinglu, Xuzhou, Jiangsu 221000 (CN); XU, Congcong, Xuzhou, Jiangsu 221000 (CN); XU, Xiangqing, Xuzhou, Jiangsu 221000 (CN)
(74) Representative: Lavoix
(86) International application number: PCT/CN2024/088722
(87) International publication number: WO 2024/217521

(57) **Abstract**

The present application relates to the field of medicines, and specifically relates to a crystalline form of a compound citrate as shown in formula I, a preparation method, and a use thereof in the field of medicines.

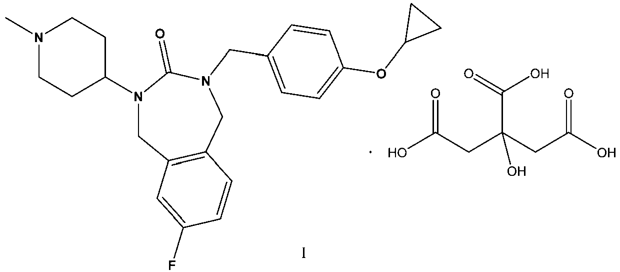

## Description

### TECHNICAL FIELD

The present application belongs to the field of medicine, and specifically relates to a crystalline form II of a citrate salt of a compound represented by formula I, a preparation method thereof, and its use in the field of medicine.

### BACKGROUND

Schizophrenia is characterized by an insidious onset, low hospitalization rate, and a high lifetime prevalence. Currently, approximately 0.3-0.7% of the global population is affected by schizophrenia in their lifetime, and in 2016, it was estimated that there were more than 21 million individuals worldwide living with schizophrenia. Currently, the primary antipsychotic drugs include typical antipsychotics and atypical antipsychotics. However, current antipsychotic drugs strongly block dopamine receptors, thus leading to adverse reactions such as extrapyramidal symptoms (EPS), tardive dyskinesia, and increased prolactin. In the field of sleep disorder treatment, although multiple types of active compounds acting on different targets are available, adverse effects including high risk of addiction, drug resistance and sequelae remain unresolved problems.

Traditionally, antipsychotic drugs that exert their pharmacological effects by blocking dopamine D₂ receptors are referred to as first-generation antipsychotics, also known as "typical" antipsychotics (such as haloperidol). They represent a breakthrough in the treatment of positive symptoms of schizophrenia, but they are ineffective against negative symptoms and cognitive impairment. Typical antipsychotics are generally associated with severe EPS side effects and are ineffective in one-third of patients with schizophrenia.

Since the 1960s, a series of new-generation antipsychotic drugs have been successively developed, including Ziprasidone, Risperidone, etc., which are called second-generation antipsychotics, also known as new antipsychotics. Although their respective pharmacological profiles are not entirely identical, they share a common pharmacological characteristic: a significantly higher affinity for 5-hydroxytryptamine (5-HT) receptors (5-HT1A, 2A, 2c) and norepinephrine (NA) receptors (α1, α2) than for D₂ receptors, resulting in a higher D₂/5-HT_{2A} ratio. Compared with first-generation antipsychotics, second- generation antipsychotics demonstrate superior clinical efficacy. They are not only as effective as traditional antipsychotics against positive symptoms, but also effective against negative symptoms and cognitive deficits, resulting in a broader spectrum of therapeutic action. However, these drugs have adverse effects such as QT interval prolongation, hyperprolactinemia and weight gain. Therefore, the development of novel drugs that are effective against positive symptoms, negative symptoms and cognitive impairment in schizophrenia, while minimizing side effects, represents a major focus of current research.

The 5-hydroxytryptamine (5-HT) system plays an important role in regulating functions of the prefrontal cortex (PFC), including emotional control, cognitive behavior, and working memory. Pyramidal neurons in the PFC and GABA interneurons contain several 5-HT receptor subtypes with particularly high densities, including 5-HT_{1A} and 5-HT_{2A}. It has recently been demonstrated that PFC and NMDA receptor channels are targets of 5-HT_{1A}R, both of which regulate excitatory neurons in the cerebral cortex, thereby affecting cognitive function. Indeed, various preclinical data suggest that the 5-HT_{1A}R may be a novel drug target for the development of antipsychotic drugs. The high affinity of atypical antipsychotics (such as olanzapine, aripiprazole, etc.) for 5-HT_{1A}R and their low EPS side effects collectively indicate that the 5-HT system plays an important role in regulating prefrontal cortex (PFC) functions, including emotional control, cognitive behavior and working memory. Pyramidal neurons in the PFC and GABA interneurons contain several 5-HT receptor subtypes, among which 5-HT_{1A} and 5-HT_{2A} are particularly abundant. Recent studies have shown that 5-HT_{1A} agonists are associated with atypical antipsychotic treatment and can improve negative symptoms and cognitive impairment. In the treatment of schizophrenia with the atypical antipsychotic clozapine, 5-HT_{2A} has been found to play a very important role, influencing various aspects including perception, emotion regulation and motor control. Blockade of 5-HT_{2A} receptors can normalize dopamine release and exert an antipsychotic effect. In addition, 5-HT_{2C} receptors are closely related to weight gain.

Pimavanserin is an inverse agonist with high affinity for 5-HT_{2A} and 5-HT_{2C}. *Ex vivo* experimental results show that its affinity for 5-HT_{2A} receptor [inhibition constant (Ki) is 0.4nM] is higher than that for 5-HT_{2C} (Ki=16nM), and it has no significant affinity (Ki>300nM) for 5-HT_{2B} receptors, dopamine receptors (including D2 receptors), adrenergic receptors, muscarinic receptors or calcium channel receptors. The drug was approved for marketing by the U.S. Food and Drug Administration in April 2016 under the brand name Nuplazid^{™}. It is mainly used to treat Parkinson's psychiatric symptoms such as hallucinations and delusions.

Patent application WO2021218863 discloses a series of 1,5-dihydro-2,4-benzodiazepine-3-one derivatives that act on 5-HT_{2A} and 5-HT_{2C} receptors. These derivatives have better selectivity for 5-HT_{2A} than Pimavanserin and are intended for the treatment of schizophrenia or behavioral disturbances and psychosis associated with Parkinson's disease and dementia. The compounds demonstrate favorable antipsychotic activity with reduced sedative side effects, less motor impairment, and lower cardiac toxicity.

For drugs, different salt forms have distinct crystalline forms, and a single salt form may also present multiple polymorphs. Different polymorphs may possess varying properties such as color, melting point, stability, apparent solubility, dissolution rate, etc. These properties directly affect the stability, solubility, hygroscopicity, and bioavailability of drug preparations, thereby leading to differences in drug quality and clinical efficacy. Therefore, it is very meaningful to develop polymorphic forms with high purity, stability or bioavailability, along with their corresponding preparation methods.

### SUMMARY

The contents disclosed in patent application WO2021218863 and Chinese patent application CN202211299097.1 are incorporated herein by reference.

The present application aims to provide a crystalline form II of the citrate salt of a compound represented by Formula I. Compared with the free base form of the compound, crystalline form II improves the stability and solubility of the active substance, reduces hygroscopicity, and enhances the bioavailability of the active substance, facilitating subsequent clinical development and production development.

The present application provides a crystalline form II of a citrate (salt) of the compound represented by formula I (hereinafter referred to as crystalline form II),

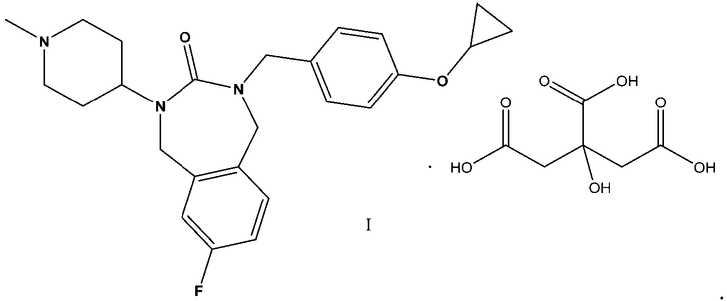

The present application also provides the use of the Crystalline form II in preparing drugs for treating neuropsychiatric diseases.

The present application further provides a method for preparing the Crystalline form II.

### DETAIL DESCRIPTION

Unless otherwise defined, all technical and scientific terms used herein have the same meaning as commonly understood by one of ordinary skill in the art to which the present application belongs. In case of conflict, the definitions provided in this application shall prevail. Where trade names are used herein, it is intended to refer to the corresponding commercial product or its active ingredient. All patents, published patent applications, and publications cited herein are incorporated herein by reference.

### General Terms and Definitions

The term "pharmaceutically acceptable carrier" refers to substances that have no significant irritation to an organism and do not impair the biological activity and properties of the active compound. "Pharmaceutically acceptable carriers" include, but are not limited to, glidants, sweeteners, diluents, preservatives, dyes/colorants, flavorings, surfactants, wetting agents, dispersants, disintegrants, stabilizers, solvents, or emulsifiers.

The "crystalline form" refers to an ordered arrangement of molecules with the same chemical structure in a certain manner.

The "X-ray powder diffraction pattern" or "XRPD" described in the present application refers to the X-ray powder diffraction pattern obtained based on Bragg equation 2d sinθ = nλ (wherein, λ is the wavelength of the X-rays, λ=1.54056Å, and n is any positive integer representing the order of diffraction, generally n = 1 for first-order diffraction peaks). When X-rays are incident at a grazing angle θ (the complementary angle of the incident angle, also known as the Bragg angle) onto a certain atomic plane with d-lattice plane spacing in a crystalline or a portion of a crystal sample, the Bragg equation is satisfied, this set of X-ray powder diffraction patterns is obtained.

The "2θ" or "20 angle" mentioned in the present application refers to the diffraction angle, wherein θ is the Bragg angle, the unit is ° or degree, and the error range of 2θ is ±0.1 to ±0.5, preferably ±0.1 to ±0.3, and more preferably ±0.2.

The term "interplanar spacing" or "interplanar spacing (d value)" mentioned in the present application refers to the selection of three non-parallel unit vectors a, b, and c connecting two adjacent lattice points in the spatial lattice. They divide the lattice into juxtaposed parallelepiped units, which are called interplanar spacing. The space lattice is divided according to the determined parallelepiped unit lines to obtain a set of straight-line grids, which are called space grids or lattices. Lattice and crystal lattice reflect the periodicity of crystal structure through geometric points and lines respectively. Different crystal planes have different interplanar spacings (that is, the distance between two adjacent parallel crystal planes); the unit is Å or angstrom.

The crystalline form protected in this application is a substantially pure crystal. As used herein, the term "substantially pure" refers to a composition comprising at least 85% by weight, preferably at least 95% by weight, more preferably at least 99% by weight, and most preferably 100% by weight, of the crystalline form disclosed herein.

"Amorphous" refers to a non-crystalline molecular and/or ionic solid form. Amorphous solids do not exhibit a sharp maximum in their X-ray diffraction patterns.

"Differential Scanning Calorimetry" or "DSC" as used herein measures the transition temperature when a crystal absorbs or releases heat due to a change in its crystal structure or melting of the crystal. For the same crystalline form of a given compound, the thermal transition temperature and melting point may vary within about 5°C in consecutive analysis, usually within about 3°C. When describing a compound as having a given DSC peak or melting point, it refers to the value within a range of ±5°C. "Essentially" also takes this temperature variation into account. DSC provides an auxiliary method to distinguish different crystalline forms. Different crystalline forms can be identified based on their distinct transition temperature characteristics. It should be noted that for mixtures, the DSC peaks or melting points may vary over a broader range. In addition, since decomposition accompanies the melting process of the substance, the melting temperature is related to the heating rate.

The term "mental disorders" refers to a neurological disorder, including schizophrenia, schizoaffective psychosis, psychosis, Parkinson's disease, behavioral and psychological symptoms of dementia (BPSD), delusional disorder, acute transient psychotic disorder, depressive disorder, bipolar disorder, generalized anxiety disorder, panic disorder, obsessive-compulsive disorder, social phobia, agoraphobia and post-traumatic stress disorder, etc.

The term "schizophrenia" refers to a group of chronic disorders with unknown etiology, which typically manifest clinically as heterogeneous syndromes involving disturbances in perception, thought, emotion and behavior, along with incoordination of mental activities. Patients are generally conscious and have basically normal intelligence, but some patients will experience cognitive impairment during the course of the disorder.

The term "psychosis" or "psychiatric disorder" refers to a disease characterized by brain dysfunction under the influence of various biological, psychological and social environmental factors, leading to varying degrees of impairment in mental activities, such as cognition, emotion, will and behavior, as clinical manifestations.

The following detailed description of the present application is intended to illustrate non-limiting embodiments, enabling other skilled in the art to more fully understand the technical solutions, principles and practical applications of the present application. This will allow other skilled in the art to modify and implement the present application in various forms to best adapt it to the requirements of specific uses.

The present application provides a crystalline form II of a citrate salt of a compound represented by formula I, characterized in that, using Cu-Kα radiation, the crystalline form II has an X-ray powder diffraction pattern with characteristic peaks represented by the following diffraction angles 2θ±0.2° at: 2.95, 5.96 and 15.09.

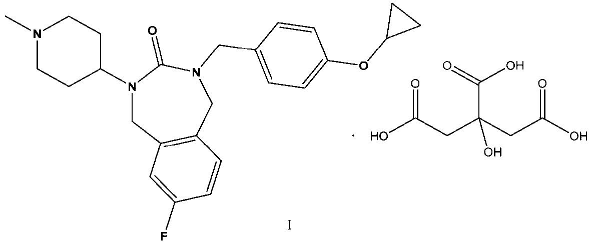

Furthermore, the X-ray powder diffraction pattern of the crystalline form II also shows one or more characteristic peaks represented by angles 2θ±0.2° at 13.96, 16.39 and 20.53, wherein each characteristic peak has a 2θ error range of ±0.2.

The X-ray powder diffraction pattern of the crystalline form II includes one or more characteristic peaks represented by angles 2θ±0.2° at the following positions: 2.95, 5.96, 10.16, 11.83, 12.28, 12.52, 13.96, 15.09, 16.14, 16.39, 18.19, 18.59, 19.09, 19.36, 20.53, 21.23, 21.93, 23.89, 26.22, 27.49, 29.44, 30.78, 35.91 and 43.33, wherein each characteristic peak has a 20 error range of ±0.2.

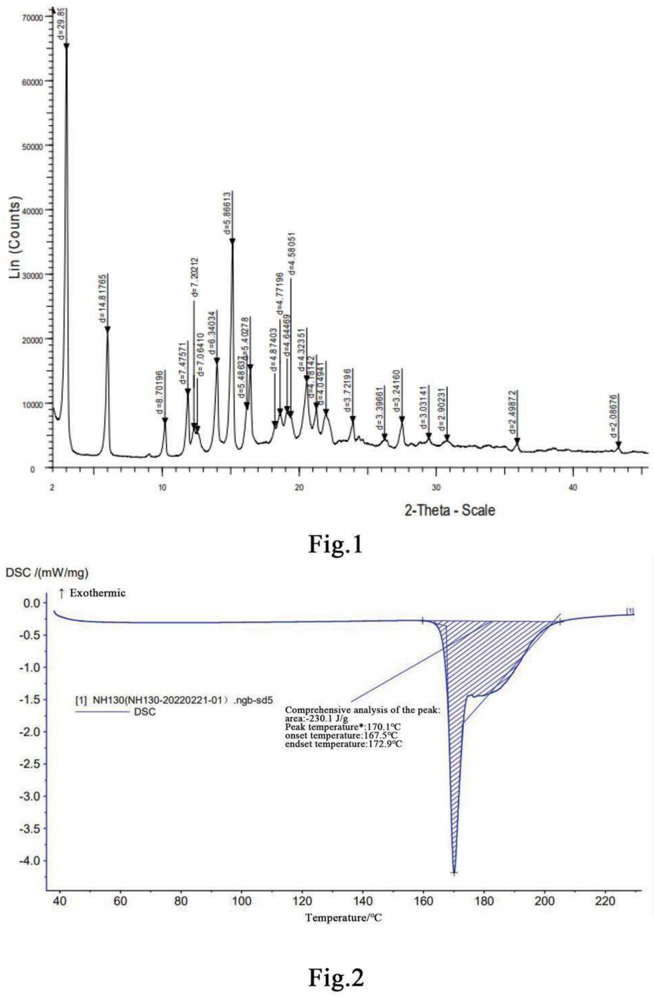

Furthermore, the X-ray powder diffraction pattern of the crystalline form II is shown in Fig. 1.

Furthermore, the melting endothermic peak of DSC for the crystalline form II is selected from about 167.5°C -about 172.9°C, preferably about 170.1°C.

The present application provides a method for preparing the above-mentioned crystalline form II, characterized by comprising the following steps:
(A) dissolving a citrate of the compound represented by Formula I in a solvent under heating until a clear solution is obtained, followed by cooling and stirring;
(B) filtering, washing, and drying to obtain crystalline form II.

In one embodiment, the solvent in step (A) is selected from methanol, ethanol, acetonitrile, tetrahydrofuran, acetone, isopropanol, n-propanol, methyl tert-butyl ether, or any combination thereof. In a preferred embodiment, the solvent in step (A) is selected from one or more of the following solvent combinations: methanol/ethanol, acetonitrile/methanol, ethanol/tetrahydrofuran, tetrahydrofuran/methanol, isopropanol/methanol, acetone/methanol, methanol, n-propanol/methanol, and methanol/methyl tert-butyl ether; in a more preferred embodiment, the solvent in step (A) is selected from one or more of the following solvent combinations: methanol/ethanol, acetonitrile/methanol, and ethanol/tetrahydrofuran.

The present application also relates to the use of the Crystalline form II in the preparation of a medicament for treating psychiatric disorders; or a method for treating a psychiatric disorder, the method comprising administering a therapeutically effective amount of the crystalline form II to a patient; or the crystalline form II for use in the treatment of psychiatric disorders.

### ADVANTAGEOUS TECHNICAL EFFECTS OF THE INVENTION

Compared with the prior art, the technical solution of the present application possesses the following advantages:
Crystalline form II exhibits favorable bioavailability and demonstrates good stability under conditions of light exposure, high temperature and high humidity. In particular, compared with the free base form of the compound, crystalline form II is beneficial for improving the stability and solubility of the active pharmaceutical ingredient, reducing the hygroscopicity, and enhancing its bioavailability. Crystalline form II exhibits good stability under light, high temperature, and high humidity conditions, which is beneficial for subsequent clinical development and production development, demonstrating promising potential for clinical application.

### BRIEF DESCRIPTION OF THE DRAWINGS

**Fig. 1** is an X-ray powder diffraction pattern of the crystalline form II of the citrate of the compound represented by Formula I;
**Fig. 2** is a DSC spectrum of the crystalline form of the citrate of the compound represented by Formula I.

### EXAMPLE

The Examples of the present application are described in detail below. The Examples described below are illustrative and are only used to explain the present application, and are not to be construed as limiting the present application. Unless otherwise specified, all ratios, percentages, etc. referred to herein are by weight.

### Synthesis Examples

### Example 1. Preparation of compound represented by general formula I

The preparation was carried out following the method described in the examples of patent application WO2021218863. 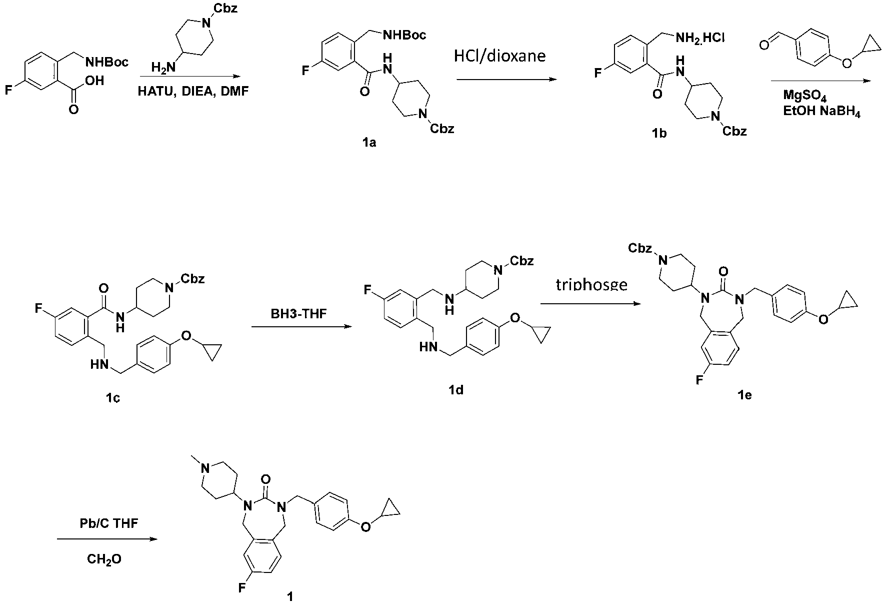
1.1: Benzyl 4-aminopiperidine-1-carboxylate (17.40 g , 74.26 mmol) and 2-[[(tert-butoxycarbonyl)amino]methyl]-5-fluorobenzoic acid were added to N,N-dimethylformamide (DMF) (200 mL) under nitrogen protection. N,N-diisopropylethylamine (DIEA) (38.40 g, 297.10 mmol) and 2-(7-azabenzotriazole)-N,N,N',N'-tetramethyluronium hexafluorophosphate (HATU) (42.36 g, 111.41 mmol) were added portion-wise at 0°C and stirred at room temperature for 2 hours. After the reaction was completed, the mixture was quenched with water at room temperature, and the mixture was extracted with EtOAc (3 x 20 mL). The organic phases were combined, washed with saturated brine (2 x 10 mL), dried over anhydrous Na₂SO₄, filtered, and the filtrate was concentrated under reduced pressure. The product was separated and purified by column chromatography (hexane/EtOAc = 6:1) to obtain 23 g of benzyl 4-[2-[[(tert-butoxycarbonyl)amino]methyl]-5-fluorobenzamido]piperidine-1-carboxylate (**1a**).
1.2: 1a (23.00 g, 47.37 mmol) was added to a DCM (200 mL) solution, under nitrogen protection, a 1,4-dioxane solution of HCl (10 mL) was added dropwise at 0°C. The mixture was stirred overnight at room temperature. After completion of the reaction, the mixture was concentrated under reduced pressure to obtain 19.1 g of benzyl 4-[2-(aminomethyl)-5-fluorobenzamido]piperidine-1-formate hydrochloride (**1b**) crude product, which was used directly in the next step without further purification.
1.3: 1b (19.00 g, 49.29 mmol) and 4-cyclopropyloxybenzaldehyde (8.8 g, 54.26 mmol) were added to an ethanol solution (200 mL) under nitrogen protection. MgSO₄ (23.70 g, 196.90 mmol) was added portion-wise at room temperature and stirred at room temperature for 30 minutes. Then NaBH₄ (4.7 g, 124.23 mmol) was added portion-wise to the above mixture at 0°C. After the addition was complete, the mixture was stirred at room temperature for 1 hour. Upon completion, the reaction was quenched with water at room temperature and extracted with EtOAc (3 x 50 mL). The organic phases were combined, washed with saturated brine (1 x 50 mL), dried over anhydrous Na₂SO₄, filtered, and the filtrate was concentrated under reduced pressure. The product was separated and purified by column chromatography (PE/EtOAc = 5:1) to obtain 13.2 g of benzyl 4-[2-[[(4-cyclopropyloxybenzyl)amino]methyl]-5-fluorobenzamido]piperidine-1-formate (**1c**).
1.4: 1c (13.00 g, 24.45 mmol) was added to THF (150 mL) under nitrogen protection. BH₃-THF (21.00 g, 244.35 mmol) was added at 0°C. After the addition was complete, the reaction mixture was warmed to 80°C and the reaction was allowed to react overnight. The mixture was then acidified to pH 5 with aqueous HCl. The resulting mixture was extracted with EtOAc (1 x 50 mL), and the pH of the aqueous layer was adjusted to 9 with saturated Na₂CO₃ solution. The resulting mixture was extracted with EtOAc (3 x 50 mL), washed with saturated brine (1 x 50 mL), dried over anhydrous Na₂SO₄, filtered, and the filtrate was concentrated under reduced pressure. The product was separated and purified by column chromatography (DCM/MeOH = 10:1) to obtain 2.3 g of benzyl 4-[[2-[[(4-cyclopropyloxybenzyl)amino]methyl]-5-fluorobenzyl]aminopiperidine-1-formate (**1d**).
1.5: 1d (2.3 g, 4.44 mmol) and triethylamine (1.80 g, 17.77 mmol) were added to THF (50 mL) under nitrogen protection, cooled to -78 °C, and triphosgene (527.40 mg, 1.78 mmol) was added in batches. The reaction was allowed to proceed at -78 °C for 1 hour. After the reaction was completed, saturated NaHCO₃ solution was added at room temperature to quench the reaction. The reaction was extracted with EtOAc (3 x 50 mL), and the organic phases were combined, washed with saturated brine (1 x 50 mL), dried over anhydrous Na₂SO₄, filtered, and the filtrate was concentrated under reduced pressure. The product was separated and purified by column chromatography (PE/EtOAc=2:1) to obtain 2.0 g of benzyl 4-[4-(4-cyclopropyloxybenzyl)-8-fluoro-3-oxo-1,3,4,5-tetrahydro-2H-benzo[e][1,3]diazepin-2-yl]pip eridine-1-formate (**1e**).
1.6: 1e (2.0 g, 3.68 mmol) and formaldehyde (220.90 mg, 9.40 mmol) were added to methanol (100 mL), and Pd/C (1.00 g, 9.40 mmol) was added portion-wise at room temperature. After the addition was complete, the reaction was placed under a hydrogen atmosphere and allowed to react at room temperature overnight. After the reaction was complete, the reaction solution was filtered, and the filtrate was concentrated under reduced pressure. The product was separated and purified by column chromatography (PE/EtOAc=1:1) to obtain 0.8 g of 2-(4-cyclopropyloxybenzyl)-7-fluoro-4-(1-methylpiperidin-4-yl)-1,2,4-tetrahydro-3H-benzo[e][1,3]d iazepine-3-one (**1**).

### Example 2. Preparation of 2-(4-cyclopropyloxybenzyl)-7-fluoro-4-(1-methylpiperidin-4-yl)-1,2,4-tetrahydro-3H-benzo[e][1 ,3]diazepine-3-one citrate

The compound was prepared according to the method described in Example 1 of patent application CN202211299097.1.

Preparation method: Approximately 10 g of the compound from Example 1 was weighed and placed into a 500 mL single-necked flask, and 200 mL of anhydrous ethanol was added. The mixture was stirred until fully dissolved. Subsequently, 5.0 g of anhydrous citric acid was weighed and dissolved in 40 mL of anhydrous ethanol. The anhydrous ethanol-citric acid solution was slowly added dropwise under stirring. During the addition, a large amount of white solid precipitated abruptly. After completion of the dropwise addition over 1 hour, the flask was transferred to an ice-water bath for cooling under continuous stirring leading to further precipitation of solid. The solid was collected by vacuum filtration and washed with anhydrous ethanol (2×40 mL). The filter cake was then vacuum-dried at 45 °C for 3 hours to obtain the citrate. Output: 12.3 g; Yield: 84.6%.¹H NMR (400 MHz, DMSO-*d*₆) δ 7.24 (d, *J =* 8.4 Hz, 2H), 7.11 - 7.21 (m, 2H), 7.00 - 7.07 (m, 1H), 6.98 (d, *J =* 8.3 Hz, 2H), 4.38 (s, 4H), 4.32 (s, 2H), 4.18 (tt, *J =* 12.2, 4.0 Hz, 1H), 3.80 (tt, *J* = 6.0, 2.9 Hz, 1H), 3.31 (d, *J =* 11.8 Hz, 2H), 2.93 - 2.78 (m, 2H), 2.66 (d, *J =* 2.8 Hz, 3H), 2.09 (qd, *J =* 13.0, 4.0 Hz, 2H), 1.66 (dd, *J =* 13.7, 3.8 Hz, 2H), 0.81 - 0.73 (m, 2H), 0.63 (q, *J =* 3.4, 3.0 Hz, 2H).

### Example 3. Preparation method of crystalline form II of 2-(4-cyclopropyloxybenzyl)-7-fluoro-4-(1-methylpiperidin-4-yl)-1,2,4,5-tetrahydro-3H-benzo[e] [1,3]diazepine-3-one citrate

### Example 3-1

A mixture of 60.0 g (97.5 mmol) of the citrate from Example 2, 170 mL of methanol, and 850 mL of anhydrous ethanol were added to a 3 L four-necked reaction flask under a nitrogen atmosphere. The mixture was heated until a clear solution was obtained, cooled, stirred at room temperature for 1 hour, and filtered. The filter cake was washed with 300 mL of anhydrous ethanol, the filtrate was discarded, and the filter cake was dried in a vacuum drying oven to obtain 55.86 g of a white solid, yielding 93.1%.

### Example 3-2

A mixture of 1.0 g (1.6 mmol) of the citrate from Example 2 and 15 mL of a mixed solvent (acetonitrile:methanol=2:1) were added to a 50 mL single-necked reaction flask. The mixture was heated until a clear solution was obtained, cooled, stirred at room temperature for 1 hour, and filtered. The filter cake was washed with 5 mL of acetonitrile, the filtrate was discarded, and the filter cake was dried in a vacuum drying oven to obtain 662 mg of a white solid with a yield of 66.2%.

### Example 3-3

1.0 g (1.6 mmol) of the citrate from Example 2 and 40 mL of a mixed solvent of anhydrous ethanol:tetrahydrofuran=3:2 were added to a 100 mL single-necked reaction flask. The mixture was heated until a clear solution was obtained, cooled, stirred at room temperature for 1 hour, further cooled in an ice bath for 1 hour, filtered, and the filtrate was discarded. The filter cake was dried in a vacuum drying oven to obtain 760 mg of a white solid with a yield of 76.0%.

The white solids obtained from Examples 3-1, 3-2 and 3-3 were respectively placed in an agate mortar, ground into fine powder, mixed uniformly, and then passed through a 100-mesh sieve to prepare test samples. Powder X-ray diffraction analysis was performed with a scanning range of 2° to 60°, confirming that all samples shared the same crystalline form, which was designated as crystalline form II. The powder X-ray diffraction pattern is shown in Fig. 1; separately, the white solids obtained from Examples 3-1, 3-2 and 3-3 were accurately weighed (3.0 to 6.0 mg) and placed in an aluminum crucible to prepare test samples. Thermal analysis was conducted using a heating rate of 10 K/min over a temperature range of 35 to 230°C. The DSC spectrum is shown in Fig. 2. During the DSC heating process, an endothermic peak was observed with an onset temperature of 167.5°C, an endset temperature of 172.9°C, and a peak temperature of 170.1°C. The characteristic peak positions of the crystalline form II are shown in Table 1 below:

**Table 1. X-ray powder diffraction**

| Angle 2θ(°) | Strength (%) |
|---|---|
| 2.95 | 100.0 |
| 5.96 | 32.1 |
| 10.16 | 10.2 |
| 11.83 | 17.2 |
| 12.28 | 8.9 |
| 12.52 | 8.2 |
| 13.96 | 24.6 |
| 15.09 | 53.2 |
| 16.14 | 13.9 |
| 16.39 | 23.0 |
| 18.19 | 9.4 |
| 18.59 | 12.4 |
| 19.09 | 13.0 |
| 19.36 | 11.9 |
| 20.53 | 20.3 |
| 21.23 | 13.8 |
| 21.93 | 12.3 |
| 23.89 | 10.6 |
| 26.22 | 6.4 |
| 27.49 | 10.5 |
| 29.44 | 6.6 |
| 30.78 | 6.2 |
| 35.91 | 5.3 |
| 43.33 | 4.5 |

### Biological test example

### Test Example 1: Stability test

### 1-1 Experimental method:

### (1) High humidity experiment

An appropriate amount of the crystalline form II and free base samples obtained from Examples 3-1, 3-2, and 3-3 above were placed in clean Petri dishes. These samples were stored under two different conditions: (1) relative humidity of 92.5% at 25°C, and (2) relative humidity of 75% ± 1% at room temperature; The samples were maintained under these conditions for 10 days. Sampling was performed on day 0 and day 10, followed by HPLC analysis.

### (2) Photostability Testing

An appropriate amount of crystalline form II and the base compound were placed in a clean Petri dish, spread evenly, and exposed in a light chamber (illuminance: 5000 lx ± 500 lx; UV intensity: 90 µW/cm²>) for 10 days. HPLC analysis was performed on day 0 and day 10.

### (3) High temperature experiment

An appropriate amount of crystalline form II and the base compound were placed in a clean Petri dish and stored at 40°C and 60°C for 10 days. HPLC analysis was performed on day 0 and day 10.

### (4) High temperature stress experiment

An appropriate amount of crystalline form II and the base compound were placed in a clean Petri dish and stored at 105°C for 24 hours. Samples were collected and subjected to HPLC analysis.

### 1-2 Experimental results: See Table 2 for the specific experimental results.

**Table 2. HPLC determination results of the stability of crystalline form II**

| Compound name | Crystalline form II | Base compound |
|---|---|---|
| original sample (0 day) | 99.839 % | 98.018 % |
| High temperature of 40 °C for 10 days | 99.756 % | 95.418 % |
| High temperature of 60 °C for 10 days | 99.723 % | 82.682 % |
| High temperature of 105 °C for 1 day | 99.709 % | 76.929 % |
| Light exposure for 10 days | 99.762 % | 95.604 % |
| 75% High humidity for 10 days | 99.749 % | 97.643 % |
| 92.5% High humidity for 10 days | 99.735 % | 97.703 % |

| | | |
|---|---|---|
| Conclusion: Crystalline form II has better stability than the base compound. | | |

### Test Example 2. Solubility Experiment

### Determination method

An appropriate amount of the test substance was accurately weighed And placed under conditions of 25°C ± 2°C. Different solvents were added in suitable quantities, and the mixture was vigorously shaken for 30 seconds every 5 minutes, and the dissolution status was observed over a period of 30 minutes. The substance was considered completely dissolved if no visual stratification between the solvent and solute was observed.

**Table 3. Solubility of Crystalline form II**

| Sample Name | solvent | sample weight (mg) | Amount used (ml) | Phenomenon within 30 mins | Conclusion |
|---|---|---|---|---|---|
| Crystalline form I I | Water | 100.17 | 100 | Not completely dissolved | Very Slightly Soluble |
| | | 100.07 | 1000 | completely dissolved | |
| Base compound | Water | 100.10 | 1000 | Not completely dissolved | insoluble |

| | | | | | |
|---|---|---|---|---|---|
| Conclusion: Crystalline form II is very slightly soluble in water; base compound is insoluble in water. | | | | | |

### Test Example 3. Pharmacokinetics and Bioavailability Test

The pharmacokinetic characteristics and bioavailability of the base compound and crystalline form II in beagle dogs were investigated.

### 1.2 Experimental equipment and reagents

| **Name** | **Model** | **Manufacturer** |
|---|---|---|
| Sciex triple quadrupole liquid chromatography-mass spectrometer | 5500+ | SCIEX |
| Mettler Toledo analytical balance | XSE105 | Mettler-Toledo Group |
| Desktop High speed refrigerated centrifuge | TL-18M | Shanghai Centrifuge Institute Co., Ltd. |
| Eppendorf micropipette | 20/200/1000 µL | Eppendorf, Germany |
| Digital display constant temperature shaker | SHA-B(A) | Jintan Medical Instrument Factory |
| Desktop ultrasonic cleaner | CPX8800H | Emerson Electric Co., USA |
| Vortex oscillator | Vortex genie 2 | Scientific Industries of the US |
| Acetonitrile | Chromatographic purity grade | TEDIA Company, USA |
| Methanol | Chromatographic purity grade | TEDIA Company, USA |
| Watsons Pure Water | 600 mL | Watsons Group |
| Formic acid | 095324 | MEDRAD, USA |

### 1.3 Experimental methods and data processing

Six healthy male beagle dogs were randomly divided into three groups, with two dogs in each group. One group received an intravenous injection of citrate (1 mg/kg), and the other two groups were orally administered capsules containing 5 mg/kg (calculated as free base) of the base form and crystalline form II, respectively. Blood samples were collected from the forelimb vein of the dogs before and at various time points after administration. Plasma drug concentrations at different times were measured by LC-MS/MS. Pharmacokinetic parameters and the absolute oral bioavailability were calculated using WinNonlin7.0 software. The specific experimental results are shown in Table 4.

**Table 4**

| PK parameter | IV-1mg/kg citrate | PO-5mg/kg | |
|---|---|---|---|
| | | base compound | crystalline form II |
| Cmax(ng/mL) | 138.1 | 10.2 | 38.8 |
| AUC₀₋ₗₐₛₜ(h*ng/mL) | 347.8 | 43.9 | 139.3 |
| bioavailability (%) | N/A | 2.4% | 7.4% |

It can be seen that after being formulated into an oral solid dosage form, the bioavailability of crystalline form II is increased by more than 3 times compared with the base compound.

### Test Example 4. Pharmacokinetics and Bioavailability Test

The pharmacokinetic characteristics and bioavailability of crystalline form II were investigated in beagle dogs.

**Experimental method:** Six healthy beagle dogs were randomly divided into two groups, with two dogs (one female and one male) in the first group, and four dogs (two females and two males) in the second group. The first group received an intravenous injection of 0.69 mg/kg (calculated based on free base) citrate, and the second group was orally administered 10 mg/kg (calculated based on free base) of crystalline form II by capsule. Blood samples were collected from the forelimb veins of beagle dogs at various time points before and after administration. Plasma was obtained by centrifugation, and drug concentrations in plasma at different time points were measured using LC-MS/MS. Pharmacokinetic parameters and the absolute oral bioavailability were calculated using WinNonlin8.0 software.

**Experimental results:** The results showed that crystalline form II was rapidly absorbed in beagle dogs after administration, with a short time to reach peak concentration and high bioavailability.

**Table 5**

| Parameters | IV-0.69mg/kg | PO-10mg/kg (crystalline form II) |
|---|---|---|
| Rsq_adj | 1.00 | 1.00 |
| T1/2 (h) | 2.20 | 3.96 |
| Tmax(h) | 0.08 | 2.25 |
| Cmax(ng/mL) | 219.8 | 754.0 |
| AUC0-last(h*ng/mL) | 446.9 | 6276.9 |
| AUC0-inf(h*ng/mL) | 466.1 | 6402.4 |
| AUC_%Extrap(%) | 4.20 | 2.12 |
| Cl (L/h/kg) | 1.48 | NA |
| Vdss(L/kg) | 2.50 | NA |
| MPT0-last(h) | 4.41 | 6.62 |
| Bioavailability% | NA | 94.8% |

| | | |
|---|---|---|
| NA: Not applicable | | |

It will be apparent to those skilled in the art that numerous modifications and variations of the present application can be made without departing from its spirit and scope. The specific embodiments described herein are provided by way of illustration only and are not intended to limit the present application in any way. The true scope of the present application is defined by the appended claims, while the Specification and Examples are to be considered as exemplary only.

## Claims

1. A crystalline form II of a citrate salt of the compound represented by formula I, having an X-ray powder diffraction pattern with characteristic peaks represented by the following diffraction angles 2θ±0.2°: 2.95, 5.96 and 15.09, using Cu-Kα radiation,

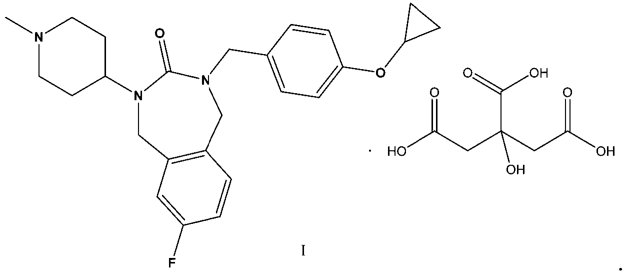

2. The crystalline form II of claim 1, wherein its X-ray powder diffraction pattern further comprises one or more characteristic peaks at 13.96, 16.39 and 20.53, wherein each characteristic peak has a 20 error range of ±0.2.

3. The crystalline form II of claim 1, wherein the X-ray powder diffraction pattern of the crystal form II is substantially as shown in Fig. 1.

4. The crystalline form II of any one of claims 1 to 3, wherein the crystalline form II has a DSC graph with an endothermic melting peak selected from about 167.5 °C to about 172.9 °C.

5. The citrate salt of claim 4, wherein the melting endothermic peak of the DSC graph of the crystalline form II is about 170.1 °C.

6. A pharmaceutical composition, comprising the crystalline form II of any one of claims 1 to 5 as an active ingredient, and a pharmaceutically acceptable carrier.

7. Use of the crystalline form II of any one of claims 1 to 5 or the pharmaceutical composition of claim 6 in the preparation of a medicament for treating psychiatric disorders.

8. The use of claim 7, wherein the psychiatric disorders are schizophrenia, psychosis, schizoaffective psychosis, Parkinson's disease, behavioral and psychological symptoms of dementia, delusional disorder, acute transient psychotic disorder, depressive disorder, bipolar disorder, generalized anxiety disorder, panic disorder, obsessive-compulsive disorder, social phobia, agoraphobia, or post-traumatic stress disorder; preferably Parkinson's disease, behavioral and psychological symptoms of dementia, or schizophrenia.

9. The preparation method of crystalline form II of any one of claims 1 to 5, comprising the following steps:
(A) dissolving a citrate of the compound represented by Formula I in a solvent under heating until a clear solution is obtained, followed by cooling, and stirring;
(B) filtering, washing, and drying to obtain crystalline form II.

10. The preparation method of crystalline form II of claim 9, wherein the solvent in step (A) is selected from: methanol, ethanol, acetonitrile, tetrahydrofuran, acetone, isopropanol, n-propanol, methyl tert-butyl ether, or any combination thereof.

11. The preparation method of crystalline form II of claim 9, wherein the solvent in step (A) is selected from one or more of the following solvent combinations: methanol/ethanol, acetonitrile/methanol, ethanol/tetrahydrofuran, tetrahydrofuran/methanol, isopropanol/methanol, acetone/methanol, methanol, n-propanol/methanol, and methanol/methyl tert-butyl ether.

12. The preparation method of crystalline form II of claim 9, wherein the solvent in step (A) is selected from one or more of the following solvent combinations: methanol/ethanol, acetonitrile/methanol, and ethanol/tetrahydrofuran.
